# EUROPEAN PATENT APPLICATION

(11) **EP 3 399 043 A1**
(43) Date of publication of application: **07.11.2018**
(21) Application number: 17733939.7
(22) Date of filing: 02.01.2017
(51) Int. Cl.: C12P 19/02, C12N 15/70, C12N 9/44

(54) **METHOD FOR PREPARING GLUCOSE FROM STARCH SUGAR BY USINGACIDOTHERMUS**

(30) Priority: 31.12.2015 KR 20150191033
(71) Applicant: Cj Cheiljedang Corporation, Seoul 04560 (KR)
(72) Inventor: AN, Jungap, Suwon-si Gyeonggi-do 16595 (KR); YOON, Ran Young, Suwon-si Gyeonggi-do 16595 (KR); KIM, Seong Bo, Seongnam-si Gyeonggi-do 13567 (KR); PARK, Seung Won, Yongin-si Gyeonggi-do 16894 (KR)
(74) Representative: Cabinet Beau de Loménie
(86) International application number: PCT/KR2017/000030
(87) International publication number: WO 2017/116217

(57) **Abstract**

The present application relates to improving the production yield of glucose to be prepared by using starch sugar as a raw material and using an *Acidothermus sp.* derived enzyme. The present application can replace an acid saccharification method, which produces a bitter taste through a hydrolysis reverse reaction, and, compared to a enzymatic saccharification method in which a conventional debranching enzyme is used, has an advantage of enabling costs to be reduced since a relatively high yield can be ensured by reducing side reactions of a saccharification reaction.

<110>	CJ CHEILJEDANG CORPORATION

<120>	Method for production of glucose from starch using debranching
	enzyme derived from Acidothermus sp. having characteristic of
	thermostability and acid-resistance, and the glucose thereof

<130>	P16-6213

<150>	KR 2015/0191033
<151>	2015-12-31

<160>	2

<170>	KopatentIn 2.0

<210>	1
<211>	2139
<212>	DNA
<213>	DNA sequece for encoding isoamylase derived from Acidothermus
cellulolyticus ATCC 43068

<400>	1
atgccggaac cgacgacgca atccctcgag gtctggccgg gcgatccgta tccgctcggt	60

gccacgtatg acggggcggg cacgaacttc gccgttttct ccgaggtcgc cgagcagatc	120

gagctctgcc tcttcgacga cgacgggaat gagactcggg tcaccctccc ggagtacgac	180

gcattcgtct ggcacggcta cctgccgggg atcggcccgg ggacacggta cgggttccgc	240

gtgcacggcc cctatgaccc cgcccgcggt ctgcggtgca atccggcgaa actcctcctc	300

gacccatacg ccaaagccat cgacggggac attgacggtc acgaatcgct cttcggttac	360

cgattcggcg atccggcgag ccgcaacgat gaagactccg cgccgcacat gatgaagtcg	420

gttgtcatca atccgttctt cgactggcgc aatgaccatc cgctccgcac gccgtaccac	480

gagtcgatca tttacgaggc gcacgtgcgc ggcatgacga tgacgcaccc ggagattccc	540

gagcatttgc gcggtacgta cgccgcgctc gcgcacccgg tgatgctcga ctacctgtct	600

tccctgggag tgacggccgt cgagctgatg ccggtgcatc aattcgtcac cgataccgcg	660

ctgcgggagc gcggcctgcg caattactgg ggatacaaca cgatcggctt ctttgccccg	720

cacaacggat attccgcatc cggcagtaga ggcgagcagg tgcaggagtt caaggccatg	780

gtccgcgccc tgcatgaagc cggtatcgag gtgatcctgg acgtcgtcta taaccacact	840

gctgaaggca atcacctcgg gcctaccctc tcattccgcg gcctggacaa cgcggcgtat	900

taccgcctgg cggatgacga tcccagccgg tacgtcgact acaccggcac cggtaacagt	960

ttcaacgccc gcaatccgca tgccttgcag ctcatcatgg attccctccg ttactggatt	1020

ctcgagatgc acgtcgacgg gttccggttc gaccttgcgt ccgcgttggc ccgtgaattg	1080

cacgacgtcg accggctctc ggccttcttc gacctcgtac agcaggatcc ggtggtgagc	1140

caggtcaagc tcatcgccga gccgtgggac gtcggcgagg gcggttacca ggtcggcaat	1200

ttcccgccgc tgtggagcga gtggaacggt aaataccgcg acacggtgcg ggatttctgg	1260

cgcggtgagc cggcgactct cggtgaattc gcctcccggc tgaccggctc ctcggacctg 1320

tacgcgagct cgggccgccg tccgatggcg tccatcaact tcgtcacctg ccacgacggc 1380

ttcaccttgc acgacctggt ctcgtacaac gagaagcaca acgaggccaa cggtgaggga 1440

aaccgtgacg gcagcgacga caatcggtca tggaactgcg gtgtcgaagg acccacggac 1500

gacgtgcaca tcatcgcgct tcgggagcaa caaaagcgca atttcctcac gacgctcctg 1560

ctctcgcagg gcgttcccat gattctgcac ggggacgagt tcgggcgaac ccaacggggc 1620

aacaacaacg cctactgcca ggacaacgaa atctcctgga tggattggcg gctcgcagtc 1680

gagcacgagg tgcaactcag cttcacccga aagctcacca cgttccggaa agaacacccg 1740

gtcttccgcc ggcggcggtt cttcgacggc aagccggttc cgcatgtcgc cggcgaggca 1800

ctgccggata tcgcctggtt caccccggcc gcggcgctga tgaccgagac ggactgggag 1860

accgggtatg ccaagagcct gaccgtcttc gtcaacggcg atgcgatccc gtcacccgac 1920

cgccgcggcc agccggtgcg ggacgattcc ttcctgttgc tgttcaacgc cgacgcgaac 1980

gacctcgaat tccgccttcc cgacgaggag tacggccagc gatgggaggc cgtcatcgac 2040

accaccgatc ccctgctcat cgatcccccg acgtacaagg cacaggcggc ggtcaccgtg 2100

ccggctcgat gcgttctggt gctgcgccgt gtcctctaa 2139

<210> 2
<211> 712
<212> PRT
<213> Isoamylase derived from Acidothermus cellulolyticus ATCC 43068

<400> 2
Met Pro Glu Pro Thr Thr Gln Ser Leu Glu Val Trp Pro Gly Asp Pro
1               5                   10                  15

Tyr Pro Leu Gly Ala Thr Tyr Asp Gly Ala Gly Thr Asn Phe Ala Val
            20                  25                  30

Phe Ser Glu Val Ala Glu Gln Ile Glu Leu Cys Leu Phe Asp Asp Asp
        35                  40                  45

Gly Asn Glu Thr Arg Val Thr Leu Pro Glu Tyr Asp Ala Phe Val Trp
    50                  55                  60

His Gly Tyr Leu Pro Gly Ile Gly Pro Gly Thr Arg Tyr Gly Phe Arg
65                  70                  75                  80

Val His Gly Pro Tyr Asp Pro Ala Arg Gly Leu Arg Cys Asn Pro Ala
                85                  90                  95

Lys Leu Leu Leu Asp Pro Tyr Ala Lys Ala Ile Asp Gly Asp Ile Asp
            100                 105                 110

Gly His Glu Ser Leu Phe Gly Tyr Arg Phe Gly Asp Pro Ala Ser Arg
        115                 120                 125

Asn Asp Glu Asp Ser Ala Pro His Met Met Lys Ser Val Val Ile Asn

130                 135                 140

Pro Phe Phe Asp Trp Arg Asn Asp His Pro Leu Arg Thr Pro Tyr His
145                 150                 155                 160

Glu Ser Ile Ile Tyr Glu Ala His Val Arg Gly Met Thr Met Thr His
                165                 170                 175

Pro Glu Ile Pro Glu His Leu Arg Gly Thr Tyr Ala Ala Leu Ala His
            180                 185                 190

Pro Val Met Leu Asp Tyr Leu Ser Ser Leu Gly Val Thr Ala Val Glu
        195                 200                 205

Leu Met Pro Val His Gln Phe Val Thr Asp Thr Ala Leu Arg Glu Arg
    210                 215                 220

Gly Leu Arg Asn Tyr Trp Gly Tyr Asn Thr Ile Gly Phe Phe Ala Pro
225                 230                 235                 240

His Asn Gly Tyr Ser Ala Ser Gly Ser Arg Gly Glu Gln Val Gln Glu
                245                 250                 255

Phe Lys Ala Met Val Arg Ala Leu His Glu Ala Gly Ile Glu Val Ile
            260                 265                 270

Leu Asp Val Val Tyr Asn His Thr Ala Glu Gly Asn His Leu Gly Pro
        275                 280                 285

Thr Leu Ser Phe Arg Gly Leu Asp Asn Ala Ala Tyr Tyr Arg Leu Ala
    290                 295                 300

Asp Asp Asp Pro Ser Arg Tyr Val Asp Tyr Thr Gly Thr Gly Asn Ser
305                 310                 315                 320

Phe Asn Ala Arg Asn Pro His Ala Leu Gln Leu Ile Met Asp Ser Leu
                325                 330                 335

Arg Tyr Trp Ile Leu Glu Met His Val Asp Gly Phe Arg Phe Asp Leu
            340                 345                 350

Ala Ser Ala Leu Ala Arg Glu Leu His Asp Val Asp Arg Leu Ser Ala
        355                 360                 365

Phe Phe Asp Leu Val Gln Gln Asp Pro Val Val Ser Gln Val Lys Leu
    370                 375                 380

Ile Ala Glu Pro Trp Asp Val Gly Glu Gly Gly Tyr Gln Val Gly Asn
385                 390                 395                 400

Phe Pro Pro Leu Trp Ser Glu Trp Asn Gly Lys Tyr Arg Asp Thr Val
                405                 410                 415

Arg Asp Phe Trp Arg Gly Glu Pro Ala Thr Leu Gly Glu Phe Ala Ser
            420                 425                 430

Arg Leu Thr Gly Ser Ser Asp Leu Tyr Ala Ser Ser Gly Arg Arg Pro
        435                 440                 445

Met Ala Ser Ile Asn Phe Val Thr Cys His Asp Gly Phe Thr Leu His
    450                 455                 460

Asp Leu Val Ser Tyr Asn Glu Lys His Asn Glu Ala Asn Gly Glu Gly

465                 470                 475                 480

Asn Arg Asp Gly Ser Asp Asp Asn Arg Ser Trp Asn Cys Gly Val Glu
                485                 490                 495

Gly Pro Thr Asp Asp Val His Ile Ile Ala Leu Arg Glu Gln Gln Lys
            500                 505                 510

Arg Asn Phe Leu Thr Thr Leu Leu Leu Ser Gln Gly Val Pro Met Ile
        515                 520                 525

Leu His Gly Asp Glu Phe Gly Arg Thr Gln Arg Gly Asn Asn Asn Ala
    530                 535                 540

Tyr Cys Gln Asp Asn Glu Ile Ser Trp Met Asp Trp Arg Leu Ala Val
545                 550                 555                 560

Glu His Glu Val Gln Leu Ser Phe Thr Arg Lys Leu Thr Thr Phe Arg
                565                 570                 575

Lys Glu His Pro Val Phe Arg Arg Arg Arg Phe Phe Asp Gly Lys Pro
            580                 585                 590

Val Pro His Val Ala Gly Glu Ala Leu Pro Asp Ile Ala Trp Phe Thr
        595                 600                 605

Pro Ala Ala Ala Leu Met Thr Glu Thr Asp Trp Glu Thr Gly Tyr Ala
    610                 615                 620

Lys Ser Leu Thr Val Phe Val Asn Gly Asp Ala Ile Pro Ser Pro Asp
625                 630                 635                 640

Arg Arg Gly Gln Pro Val Arg Asp Asp Ser Phe Leu Leu Leu Phe Asn
                645                 650                 655

Ala Asp Ala Asn Asp Leu Glu Phe Arg Leu Pro Asp Glu Glu Tyr Gly
            660                 665                 670

Gln Arg Trp Glu Ala Val Ile Asp Thr Thr Asp Pro Leu Leu Ile Asp
        675                 680                 685

Pro Pro Thr Tyr Lys Ala Gln Ala Ala Val Thr Val Pro Ala Arg Cys
    690                 695                 700

Val Leu Val Leu Arg Arg Val Leu
705                 710

## Description

### [Technical Field]

The present invention relates to a method for producing glucose from starch sugars using a debranching enzyme derived from an *Acidothermus sp.* and a diastatic enzyme.

### [Background Art]

Biomass can be broadly divided into sugar biomass (sugarcane), starchy biomass (corn and sweet potato), and cellulosic biomass (wood and silver grass) according to major ingredients. Sugars are easily obtained from sugarcane and can be directly ingested by organisms, avoiding the need for additional pretreatment or glycosylation. However, starchy biomass such as corn biomass is not directly available to microorganisms due to the high molecular weight of starch. For this reason, starch needs to be broken down into glucose. This course requires water and is thus called "hydrolysis".

Starch is hydrolyzed mainly by enzymes, just as we digest rice. Cooked rice we eat is degraded into glucose in the stomach by enzymes secreted from salivary glands, and the glucose is transferred to body cells and is then used for metabolic processes. The first process for converting starch to glucose is to make the structure of starch loose, just as we cook rice by heating, with the result that enzymes can approach the starch and break it down to glucose. This enzymatic degradation process consists of inherently complex mechanisms.

An enzymatic glycosylation is a process for converting starch to glucose. According to this process, starch is liquefied by an alpha-amylase derived from a microorganism and the liquefied starch reacts with a debranching enzyme and a diastatic enzyme to produce glucose.

### [Disclosure]

### [Technical Problem]

Promozyme® (Novozyme) is a pullulanase isolated from a *Pseudomonas sp.* and has been mainly used as a debranching enzyme for enzymatic glycosylation. However, due to its specificity for starch sugars with a narrow range of degrees of polymerization (DP 4-5), Promozyme® is unsuitable for the hydrolysis of branched chains of various lengths (DP 3-30) present in starch sugars, resulting in low glucose yield.

### [Technical Solution]

The present invention relates to a method for producing glucose from starch sugars using a novel debranching enzyme, and specifically, a method for producing glucose, which includes simultaneously adding a debranching enzyme derived from a heat-resistant and acid-tolerant *Acidothermus sp.* and a diastatic enzyme to a substrate solution containing one or more starch sugars selected from the group consisting of starch, amylopectin, and maltodextrin to react the enzymes with the starch sugars.

More specifically, starch sugars are treated with an isoamylase as a debranching enzyme derived from an *Acidothermus sp.* to hydrolyze branched chains of various lengths present therein, enabling the production of glucose from the starch sugars in high yield.

It is another object of the present invention to provide a method for producing glucose from starch sugars in high yield using an isoamylase derived from an *Acidothermus sp.* and a diastatic enzyme in a predetermined unit ratio.

In order to achieve the above and other objects of the present invention, one aspect of the present invention provides a method for producing glucose from starch sugars, including treating a substrate solution containing starch sugars with a debranching enzyme derived from an *Acidothermus sp.* and a diastatic enzyme.

Specifically, the debranching enzyme derived from an *Acidothermus sp.* may be produced by a method including inserting a gene encoding the debranching enzyme into a vector to produce an expression vector, transforming the expression vector into cells, and culturing the transformed cells. The method may further include centrifuging and/or disrupting the resulting culture. The culture can be disrupted by any suitable technique known in the art, for example, using an ultrasonic homogenizer.

The vector may be pET-28a(+).

The debranching enzyme derived from an *Acidothermus sp.* may be an isoamylase. The debranching enzyme has hydrolytic activity on α-1,6-bonds, particularly specificity for starch sugars with a wide range of degrees of polymerization(DP3-30), resulting in an increase in glucose yield.

The diastatic enzyme is an enzyme that can catalyze the hydrolysis of debranched starch sugars or starch sugars into glucose. An addition of the diastatic enzyme enables the final production of glucose from the chains of the starch sugars cleaved by the debranching enzyme. For example, the diastatic enzyme may be selected from α-amylases, β-amylases, and glucoamylases. Specifically, the diastatic enzyme may be a glucoamylase. The diastatic enzyme catalyzes glycosylation in the pH range of 3 to 7, more specifically 3 to 6. The reaction temperature of the diastatic enzyme may be from 40°C to 70°C, specifically from 55°C to 65°C.

The *Acidothermus sp.* may be *Acidothermus cellulolyticus* strain. For example, the *Acidothermus cellulolyticus* strain may be Acidothermus cellulolyticus strain 11B [ATCC® 43068™]. The said strain-derived isoamylase is encoded by the sequence set forth in SEQ ID NO. 1 and has the amino acid sequence set forth in SEQ ID NO. 2.

The cells which can be transformed with the expression vector may be, for example, any one of *E. coli,* a microorganism belonging to the genus *Bacillus,* yeast (*e.g.,* Baker's yeast), a microorganism belonging to the genus *Corynebacterium* and a plant.

The starch sugars may be any one or more selected from the group consisting of for example starch, amylopectin, and maltodextrin. Specifically, the starch sugar may be amylopectin-type starch. Amylopectin and maltodextrin may be prepared by adding an alpha-amylase to starch.

The starch sugars may be derived from any one crop selected from for example, corn, tapioca, potato, and rice, but may not be limited to that.

The starch sugars may be liquefied starch ones.

The substrate solution may be treated with the debranching enzyme derived from an *Acidothermus sp.* and the diastatic enzyme at a pH of 2 to 6, more preferably 3 to 5, most preferably 4.3.

The substrate solution may be treated with the debranching enzyme derived from an *Acidothermus sp.* and the diastatic enzyme at a temperature of 40°C to 70°C, more preferably 50°C to 65°C, most preferably 65°C.

The unit ratio of the debranching enzyme activity derived from an *Acidothermus sp.* to the diastatic enzyme activity may be the range from 2:1 to 60:1, specifically from 10:1 to 60:1, more specifically from 10:1 to 40:1.

The aid of the debranching enzyme is required to exceed the reaction equilibrium of the diastatic enzyme. The glucose yield increases in proportion to the unit ratio of enzyme activity. However, if the unit ratio of the diastatic enzyme activity to the debranching enzyme activity derived from an *Acidothermus sp.* exceed to 1:60, the glucose yield may be decreased. The diastatic enzyme plays a major role in the degradation of starch sugars to glucose (*i.e*. glycosylation) and the debranching enzyme plays an auxiliary role in glycosylation. Therefore, if the unit ratio of the debranching enzyme activity derived from an *Acidothermus sp.* to the diastatic enzyme activity is less than 2:1, the role of the diastatic enzyme in the degradation of starch sugars cannot be expected, resulting in a low glucose yield.

Another aspect of the present invention provides a method of using a debranching enzyme derived from an *Acidothermus sp.* in the production of glucose from starch sugars.

A further aspect of the present invention provides a debranching enzyme derived from Acidothermus cellulolyticus that is active at a temperature of 40°C to 70°C and a pH of 2 to 6. The debranching enzyme has a hydrolytic activity on α-1,6-bonds and good selectivity for chain lengths, particularly chain lengths corresponding to DPs of 3 and 4, 3 or more, or 4 or more, resulting in an increase in a glucose yield.

The debranching enzyme is active at a temperature of 40°C to 70°C, more preferably 50°C to 65°C, most preferably 60°C.

The debranching enzyme is active at a pH of 2 to 6, more preferably pH 3 to 5, most preferably pH 4.3.

### [Advantageous Effects]

According to the present invention, the use of the debranching enzyme (isoamylase) derived from an *Acidothermus sp.* enables the production of glucose from starch sugars in high yield compared to the use of a conventional commercial debranching enzyme (pullulanase).

In addition, the ratio between the diastatic enzyme and the debranching enzyme used in the multiple enzymatic reactions is adjusted such that the amounts of by-products(DP2) and unreacted products (DP3, DP4+) in the hydrolysis of starch sugars are reduced, resulting in an improvement in the yield of glucose from the starch sugars.

Particularly, the debranching enzyme used in the method for producing glucose from starch sugars according to the present invention maintains its activity under strong acid and/or high temperature conditions, thus greatly improving the yield of glucose.

### [Description of Drawings]

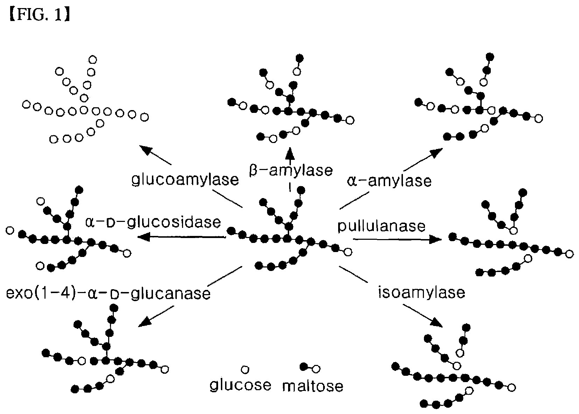

FIG. 1 shows several hydrolases and pathways for the degradation of starch sugar.

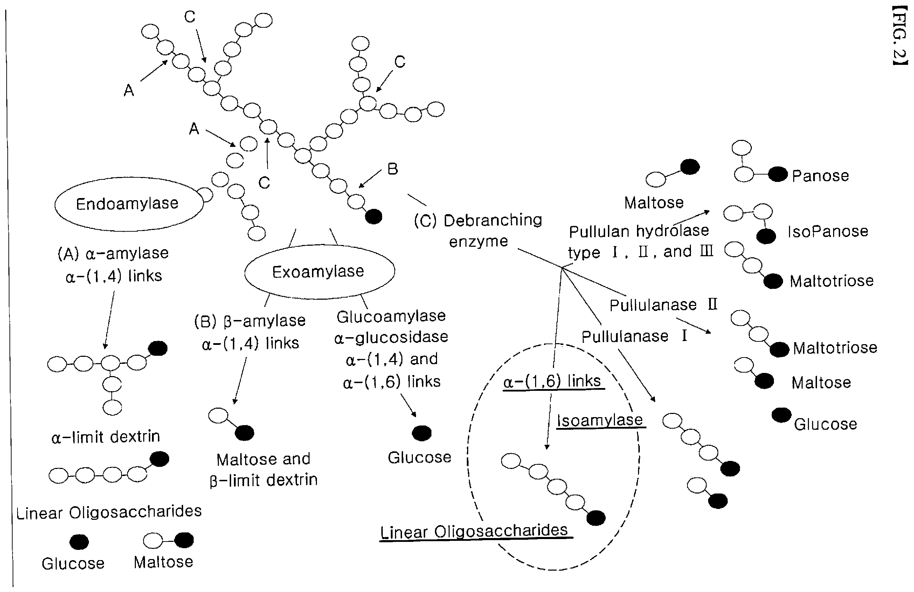

FIG. 2 shows several debranching enzymes and pathways for the degradation of starch sugar.

### [Mode for Invention]

The present invention will be described in more detail with reference to the following examples. These examples are merely illustrative to assist in understanding the invention and should not be construed as limiting the scope of the invention.

### Example 1: Gene isolation and recombinant protein production

### 1) Production of recombinant E. coli

Forward and reverse primer sequences including portions of both end sequences of a debranching enzyme gene derived from a heat-resistant and acid-tolerant *Acidothermus cellulolyticus* and the recognition sequences of restriction enzymes NdeI and HindIII, respectively, were used for PCR.

(Forward) 5'-GAA-TTC-ATG-CCG-GAA-3'
(Reverse) 5'-GAA-TTC-TTA-GAG-GAC-3'

As a result, a PCR product of 2.1 kb was obtained. The acquired DNA fragment was inserted into pET-28a(+) vector and transfected into *E. coli* BL21(DE3). The DNA has the sequence set forth in SEQ ID NO. 1. The transformed *E. coli* was spread on the plate media containing kanamycin. Kanamycin-resistant strains were primarily screened and separately cultured in liquid media. DNA of Kanamycin-resistant strain was purified and digested with both NdeI and HindIII. The finally selected strain was found to have 2.1 kb and 5.3 kb DNA fragments. The sequence was analyzed using an automatic sequencer. As a result, the sequence was found to be identical to the gene sequence of the desired debranching enzyme.

### 2) Recombinant protein production

A debranching enzyme (isoamylase) was derived from acid-tolerant and heat-resistant *Acidothermus cellulolyticus* by the following procedure.

A test tube containing 5 ml of LB liquid medium was inoculated with frozen-stored recombinant *E. coli* strain BL21(DE3). The inoculum was cultured in an incubator at 37°C until an absorbance of 2.0 at 600 nm was reached. The culture broth was added to 500 ml of LB liquid medium in a 2 L flask, followed by main culture. When the absorbance of the culture at 600 nm reached 0.4, 0.1 mM isopropyl β-D-1-thiogalactopyranoside (IPTG) was added to induce mass expression of a debranching enzyme. The temperature was maintained at 37°C with stirring at 180 rpm during culture. Even after the addition of the IPTG, the culture was continued at 20°C with stirring at 120 rpm. The culture broth of the transformed strain was centrifuged at 6,000×g and 4°C for 20 min and washed twice with 50 mM Tris-Cl buffer. Then, Ni-NTA binding buffer (50 mM sodium phosphate, 300 mM NaCl, 10 mM imidazole, pH 8.0) was added and the cell solution was disrupted using an ultrasonic homogenizer. The cell lysate was centrifuged at 13,000×g and 4°C for 20 min. The supernatant was collected as an enzyme extract and purified using a Ni-NTA superflow column to accurately characterize the enzyme. The purified enzyme was found to have a molecular weight of about-75 kDA, as determined by SDS-PAGE.

### Example 2: The measurement of an-enzymatic activity by HPLC

1) The activity of the debranching enzyme (isoamylase) derived from the acid-tolerant and heat-resistant microorganism (*Acidothermus cellulolyticus*) was measured by HPLC under the following conditions.

HPLC analysis conditions:
- Detector: RID
- Flow rate: 0.6 ml/min
- Sample injection vol.: 10 µl
- Column: Bio-Rad HPX 87C
- Solvent: D.I. Water

The total time for analysis was set to 20 min.

The activity of the debranching enzyme (isoamylase) derived from the acid-tolerant and heat-resistant microorganism (*Acidothermus cellulolyticus*) was confirmed by the production of glucose from corn starch as a raw material through multiple enzymatic reactions with a diastatic enzyme (Glucoamylase). The enzymatic reaction conditions were as follows.

First, a diastatic enzyme (Glucoamylase, DuPont company) and the debranching enzyme (isoamylase, 50 mM sodium acetate buffer, pH 4.3) produced in Example 1 were used in a total unit ratio of 1:4. Multiple enzymatic reactions with liquefied starch having a dextrose equivalent (DE) of 9.5 were performed at a temperature of 60°C for 72 h. The reactions were quenched by heating at 100°C for 5 min. Then, the amount of glucose produced was measured by HPLC. The results of HPLC analysis revealed that the yield of glucose from the corn starch was 96.2%. The glucose yield was calculated from the ratio between the areas of peaks corresponding to the enzymatic reaction products.

### Example 3: The comparison of yields of glucose produced using the debranching enzyme (isoamylase) derived from Acidothermus sp. and commercial debranching enzyme (Pullulanase)

The yields of glucose from starch using the debranching enzyme derived from *Acidothermus cellulolyticus* and a commercial debranching enzyme (Pullulanase, Novozyme company) were investigated. The enzymatic reaction conditions were as follows.

First, a diastatic enzyme (Glucoamylase, DuPont company) and each of the debranching enzymes (isoamylase and pullulanase) were used in a total unit ratio of 1:4. Multiple enzymatic reactions with liquefied starch (DE 9.5) were performed at a temperature of 60°C for 72 h. The reactions were quenched by heating at 100°C for 5 min. Then, the amounts of glucose produced were measured from the ratios between the areas of peaks corresponding to the enzymatic reaction products by HPLC. As a result, the reactions using the debranching enzyme (isoamylase) derived from Acidothermus cellulolyticus gave a higher glucose yield than the reactions using a commercial debranching enzyme (Pullulanase). Starch sugars, including di-(DP2) and higher saccharides, were analyzed. As a result, when the debranching enzyme (isoamylase) derived from Acidothermus cellulolyticus was used, the proportions of the unreacted products (DP3, DP4+) were reduced, indicating relatively high yield of glucose.

**Table 1**

| Diastatic enzyme | Debranching enzyme | Glucose (%) | DP2 (%) | DP3 (%) | DP4+(%) |
|---|---|---|---|---|---|
| Glucoamylase (DuPont company) (Ratio 1) | Isoamylase derived from *Acidothermus sp.* (Ratio 4) | 96.20 | 2.57 | 0.39 | 0.84 |
| | Pullulanase (Novozym company)(Ratio 4) | 96.10 | 2.16 | 0.64 | 1.10 |

As shown in Table 1, the use of the isoamylase derived from Acidothermus cellulolyticus in the present application led to a higher glucose yield due to its higher activity than the use of the pullulanase known to be optimal for mass production. The proportions of the unreacted products (DP3 and DP4+) with relatively high degrees of polymerization were smaller when the said isoamylase was used than when the pullulanase was used. From these results, it can be concluded that the application of the inventive isoamylase to enzymatic reactions leads to an increase in glucose yield.

### Example 4: The changes in glucose yield with varying concentrations of the debranching enzyme (isoamylase) derived from Acidothermus sp.

**The changes in** glucose yield with varying concentrations of the debranching enzyme (isoamylase) derived from Acidothermus cellulolyticus were investigated. The enzymatic reaction conditions were as follows. Multiple enzymatic reactions with liquefied starch (DE 9.5) were performed at a temperature of 60°C for 72 h, wherein a diastatic enzyme (Glucoamylase, DuPont company) and the debranching enzyme were used in total unit ratios of 1:2, 1:10, 1:20, and 1:60, respectively. The reactions were quenched by heating at 100°C for 5 min. Then, the amounts of glucose produced were measured from the ratios between the areas of peaks corresponding to the enzymatic reaction products by HPLC. As a result, the higher the ratio of the debranching enzyme (isoamylase) to the diastatic enzyme, the higher the glucose yield. These results appear to be because the higher activity of the debranching enzyme (isoamylase) derived from Acidothermus cellulolyticus leads to the production of smaller amounts of by-products (DP2) formed in the starch hydrolysis and the degradation of larger amounts of the unreacted products (DP4) by the diastatic enzyme, resulting in an increase in glucose yield.

**Table 2**

| Diastatic enzyme (glucoamylase, DuPont company) | Debranching enzyme (Isoamylase from *Acidothermus*) | Glucose(%) | DP2(%) | DP3(%) | DP4+(%) |
|---|---|---|---|---|---|
| Ratio 1 | Ratio 2 | 95.91 | 2.31 | 0.57 | 1.21 |
| | Ratio 10 | 96.71 | 2.15 | 0.60 | 0.54 |
| | Ratio 20 | 96.94 | 1.99 | 0.63 | 0.43 |
| | Ratio 60 | 97.07 | 1.90 | 0.63 | 0.40 |

As shown in Table 2, glucose was produced in high yield with the increasing total unit ratio of the diastatic enzyme to the isoamylase from 1:2 to 1:60. That is, the yield of glucose increased with the increasing ratio of the isoamylase to the diastatic enzyme.

### Example 5: The changes in glucose yield with the varying concentrations of diastatic enzyme in multiple reactions of the debranching enzyme derived from an Acidothermus sp. and the diastatic enzyme

The changes in glucose yield with the varying concentrations of a diastatic enzyme (Glucoamylase, DuPont company) in multiple reactions of the debranching enzyme (isoamylase) and the diastatic enzyme were investigated. The enzymatic reaction conditions were as follows. Multiple enzymatic reactions with liquefied starch (DE 9.5) were performed at a temperature of 60°C for 72 h, wherein the debranching enzyme and the diastatic enzyme were used in total unit ratios of 1:0.05, 1:0.04, 1:0.03, and 1:0.02, respectively. The reactions were quenched by heating at 100°C for 5 min. Then, the amounts of glucose produced were measured from the ratios between the areas of peaks corresponding to the enzymatic reaction products by HPLC. As a result, the smaller the proportion of the diastatic enzyme, the higher the glucose yield. When the diastatic enzyme (glucoamylase) in the reaction for starch hydrolysis reached the equilibrium of hydrolysis, by-products (DP2) were formed. This problem was solved by lowering the concentration of the diastatic enzyme such that the formation of by-products is reduced, resulting in an increase in the relative yield of glucose.

**Table 3**

| Debranching enzyme (Isoamylase from *Acidothermus*) | Diastolic enzyme (Glucoamylase, DuPont company) | Glucose(%) | DP2(%) | DP3(%) | DP4+(%) |
|---|---|---|---|---|---|
| Ratio 1 | Ratio 0.05 | 96.78 | 2.35 | 0.45 | 0.43 |
| | Ratio 0.04 | 96.90 | 2.08 | 0.53 | 0.49 |
| | Ratio 0.03 | 97.14 | 2.01 | 0.51 | 0.34 |
| | Ratio 0.02 | 96.25 | 2.05 | 1.22 | 0.48 |

As shown in Table 3, when the total unit ratio of the debranching enzyme to the diastatic enzyme changed from 1:0.05 to 1:0.03, the glucose yield increased. That is, the smaller the proportion of the diastatic enzyme, the higher the glucose yield.

### Comparative Example 1: The comparison of amylopectin degradation activities of isoamylases derived from various strains (Activity evaluation under the glycosylation conditions (60°C, pH 4.3))

**Table 4**

| No. | Enzyme | Strain | Activity(unit) |
|---|---|---|---|
| 1 | Isoamylase | *Enterobacter cloacae subsp. Cloacae* | 0 |
| 2 | | *Bifidobacterium longum subsp. Longum* | 0 |
| 3 | | *Thermotoga neapolitana* | 0 |
| 4 | | *Escherichia coli str. K-12 substr. W3110* | 0 |
| 5 | | *Sinorhizobium meliloti* | 0 |
| 6 | | *Acidothermus cellulolyticus* | 130.18 |
| 7 | | *Novosphingobium aromaticivorans* | 0 |
| 8 | | *Thermotoga thermarum* | 0 |
| 9 | | *Rhodothermus marinus* | 105.04 |

The amylopectin degradation activities of isoamylases derived from various strains under the glycosylation conditions were compared. The results are shown in Table 4. Only the enzymes derived from Acidothermus cellulolyticus and Rhodothermus marinus were found to be active for the degradation of amylopectin. Thereafter, the yields of glucose after treatment with the two enzymes were compared.

### Comparative Example 2: The comparison of glucose yields after treatment with the debranching enzymes (isoamylases) derived from Acidothermus cellulolyticus and Rhodothermus marinus

The yields of glucose from starch after treatment with the debranching enzymes derived from *Acidothermus cellulolyticus* and Rhodothermus marinus were investigated. The enzymatic reaction conditions were as follows.

First, Multiple enzymatic reactions with liquefied starch (DE 9.5) were performed at a temperature of 60°C for 72 h, wherein a diastatic enzyme (Glucoamylase, DuPont company) and each of the debranching enzymes (isoamylase, pullulanase) were used in total unit ratios of 1:2 to 1:60. The reactions were quenched by heating at 100°C for 5 min. Then, the amounts of glucose produced were measured from the ratios between the areas of peaks corresponding to the enzymatic reaction products by HPLC. As a result, higher glucose yields were obtained when the debranching enzyme (isoamylase) derived from *Acidothermus cellulolyticus* was used (see the results in Table 6) than when the debranching enzyme derived from *Rhodothermus marinus* was used (see the results in Table 5). In addition, starch sugars, including di- (DP2) and higher saccharides, were analyzed. As a result, when the debranching enzyme (isoamylase) derived from Acidothermus cellulolyticus was used, the proportions of the unreacted products (DP2, DP4+) which did not degrade to glucose were reduced, indicating relatively high yields of glucose.

**Table 5**

| Diastatic enzyme (Glucoamylase, DuPont company) | Debranching enzyme (Isoamylase derived from *Rhodothermus*) | Glucose(%) | DP2(%) | DP3(%) | DP4+(%) |
|---|---|---|---|---|---|
| Ratio 1 | Ratio 2 | 95.88 | 2.48 | 0.35 | 1.29 |
| | Ratio 10 | 96.26 | 2.31 | 0.37 | 1.06 |
| | Ratio 20 | 96.34 | 2.24 | 0.44 | 0.98 |
| | Ratio 60 | 96.61 | 2.08 | 0.56 | 0.75 |

**Table 6**

| Diastatic enzyme (Glucoamylase, DuPont company) | Debranching enzyme (Isoamylase derived from *Acidothermus*) | Glucose(%) | DP2(%) | DP3(%) | DP4+(%) |
|---|---|---|---|---|---|
| Ratio 1 | Ratio 2 | 95.91 | 2.31 | 0.57 | 1.21 |
| | Ratio 10 | 96.71 | 2.15 | 0.60 | 0.54 |
| | Ratio 20 | 96.94 | 1.99 | 0.63 | 0.43 |
| | Ratio 60 | 97.07 | 1.90 | 0.63 | 0.40 |

As shown in Tables 5 and 6, the higher glucose yields and smaller proportions of the unreacted products (DP2 and DP4) with relatively high degrees of polymerization were obtained when the isoamylase derived from *Acidothermus cellulolyticus* was used than when the debranching enzyme derived from Rhodothermus marinus was used. That is, the application of the inventive isoamylase to enzymatic reactions leads to an increase in glucose yield.

## Claims

1. A method for producing glucose from starch sugars, comprising: treating a substrate solution containing starch sugars with a debranching enzyme derived from a heat-resistant and acid-tolerant *Acidothermus sp.* and a diastatic enzyme.

2. The method according to claim 1, wherein the debranching enzyme derived from an *Acidothermus sp.* is produced by inserting a gene encoding the debranching enzyme into a vector to produce an expression vector, transforming the expression vector into cells, and mass-producing the transformed cells.

3. The method according to claim 2, wherein the vector is pET-28a(+).

4. The method according to claim 1, wherein the debranching enzyme derived from an *Acidothermus sp.* is an isoamylase.

5. The method according to claim 1, wherein the *Acidothermus sp.* is *Acidothermus cellulolyticus.*

6. The method according to claim 1, wherein the transformed cells are any one of *E. coli,* a microorganism belonging to the genus *Bacillus,* yeast, a microorganism belonging to the genus *Corynebacterium* and a plant.

7. The method according to claim 1, wherein the starch sugars are amylopectin-type starch.

8. The method according to claim 1, wherein the starch sugars are liquefied ones.

9. The method according to claim 1, wherein the starch sugars are derived from any one crop selected from corn, tapioca, potato, and rice.

10. The method according to claim 1, wherein the substrate solution containing starch sugars is treated with the debranching enzyme derived from an *Acidothermus sp.* and the diastatic enzyme at a pH of 2 to 6.

11. The method according to claim 1, wherein the substrate solution containing starch sugars is treated with the debranching enzyme derived from an *Acidothermus* sp.and the diastatic enzyme at a temperature of 40°C to 70°C.

12. The method according to claim 1, wherein the total unit ratio of the debranching enzyme derived from an *Acidothermus sp.* to the diastatic enzyme is from 2:1 to 60:1.

13. A method of using a debranching enzyme derived from a heat-resistant and acid-tolerant *Acidothermus sp.* in the production of glucose from starch sugars.

14. A debranching enzyme derived from *Acidothermus cellulolyticus* that is active at a temperature of 40°C to 70°C and a pH of 2 to 6.
